# EUROPEAN PATENT APPLICATION

(11) **EP 2 436 374 A1**
(43) Date of publication of application: **04.04.2012**
(21) Application number: 10184461.1
(22) Date of filing: 30.09.2010
(51) Int. Cl.: A61K 9/00, A61K 9/02, A61K 31/4178, A61K 47/14, A61K 47/02

(54) **Ovule for vaginal administration of arasertaconazole**

(71) Applicant: Ferrer Internacional, S.A., 08028 Barcelona (ES)
(72) Inventor: Santos, Benjamín, 08028, BARCELONA (ES); Baleeiro, Thaïs, 08028, BARCELONA (ES); Martí, Nuria, 08028, BARCELONA (ES)
(74) Representative: Jané Bonet, Montserrat

(57) **Abstract**

The invention relates to an ovule for vaginal administration of arasertaconazole mononitrate consisting of arasertaconazole mononitrate, colloidal silica and a fatty base.

## Description

The present invention relates to an ovule for vaginal administration of arasertaconazole mononitrate, R-(-)-1-[2-(7-chlorobenzo[b]tiophen-3-yl-methoxy)-2-(2,4-dichlorophenyl-ethyl]-1 H-imidazole mononitrate.

### BACKGROUND OF THE INVENTION

Arasertaconazole corresponds to the R-enantiomer of sertaconazole. Sertaconazole (EP151477B1) is widely used in clinical practice as an antifungal agent and the mononitrate is its preferred salt. The most active enantiomer of the two enantiomers of sertaconazole is arasertaconazole. The structural formula of arasertaconazole mononitrate is 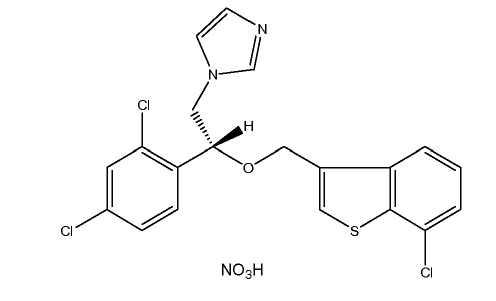 Arasertaconazole mononitrate

Sertaconazole mononitrate itself has already been formulated as an ovule for vaginal application, which is being marketed in France under the trade name of Monazol^{®}. Each Monazol^{®} ovule contains 300 mg of sertaconazole mononitrate and solid semi-synthetic glycerides and colloidal anhydrous silica as excipients. Monazol^{®} 300 mg ovule is indicated in the local treatment of infections caused by *Candida* in vaginal mucosa.

However, clinical practice has shown that there is a need to provide more potent formulations that allow greater freedom from dosing on the basis of a dose-dependent behavior. Thus, different types of pathology and potential resistances might be treated.

### SUMMARY OF THE INVENTION

The invention provides a new vaginal ovule of arasertaconazole mononitrate, which has surprisingly shown higher and more prolonged levels than when a Monazol ovule is administered. This gives rise to greater treatment efficacy, which enables management of more severe cases as well as candidiasis that may become resistant.

In addition, plasma concentrations of arasertaconazole mononitrate vaginal ovule are proportional to the dose administered. This simplifies dose adjustment to case severity.

### DETAILED DESCRIPTION OF THE INVENTION

The object of the present invention is to provide an ovule of arasertaconazole mononitrate for vaginal administration comprising an amount of 100-1000 mg of arasertaconazole mononitrate and a mixture of excipients, which comprises:
(i) colloidal silica in the range from 0.5 to 5% by weight of the entire ovule;
   and
(ii) fatty base in a sufficient range up to 100% by weight of the entire ovule.

In the present invention, the expression "fatty base in a sufficient range up to 100% by weight of the entire ovule" means that the amount of fatty base is such to get the 100% by weight of the entire ovule. The amount of fatty base will depend on the amounts of the active ingredient and colloidal silica and, if present, on the amount of additional excipients.

It is understood that the ovule for vaginal administration of the present invention may be any solid pharmaceutical dosage form at room temperature whose physical properties, such as shape, size and consistency, will facilitate its therapeutic use by vaginal insertion.

In a preferred embodiment, the proportion of arasertaconazole mononitrate represents from 3 to 50% by weight of the entire ovule.

In a preferred embodiment, the amount of arasertaconazole mononitrate comprises from 150 to 600 mg per ovule.

In a preferred embodiment, the proportion of arasertaconazole mononitrate represents from 5 to 30% by weight of the entire ovule.

In another preferred embodiment, colloidal silica is present in a proportion of 3% by weight of the entire ovule.

In another preferred embodiment, colloidal silica is in anhydrous form. Non-limitative examples of other additives, which may either replace or be mixed with colloidal anhydrous silica, include: surfactants for the purpose of favoring the release or absorption of the drug substance; consistency factors (also called hardeners), such as beeswax, cetyl alcohol, stearic acid, stearyl alcohol, aluminium monostearate, aluminium distearate, aluminium tristearate, bentonite or magnesium stearate, and mixtures thereof for the purpose of increasing melting point; plasticizers, such as glyceryl monostearate, myristyl alcohol, polysorbate 80 or propylene glycol, and mixtures thereof for the purpose of decreasing melting point; as well as the mixtures of surfactants, consistency factors and plasticizers.

In another preferred embodiment, fatty base is selected from triglyceride, monoglyceride and diglyceride esters of C₈ - C₂₀ fatty acids and the mixtures thereof. Non-limitative examples of these fatty acids include capric acid, caprylic acid, eicosenoic acid, stearic acid, lauric acid, myristic acid, oleic acid, palmitic acid, ricinoleic acid and their derivatives. Many of these conveniently formulated fatty bases, which are prepared from their natural sources (coconut oil, palm oil and the like) and optionally mixed with various additives in order to adapt them to different uses, are commercially available. Non-limitative examples of trade names for fatty bases are Suppocire^{®}, Ovucire^{®}, Japocire^{®}, Witepsol^{®}, Massa estarinum^{®}, Wecobee^{®} and Novata^{®}. It is also possible to substitute fatty bases with water-soluble bases or mix fatty bases with water-soluble bases. Non-limitative examples of water-soluble bases, which are useful in the present invention, include among others polyethylene glycols and glycerolated glycerin bases.

It is also an object of this invention to provide a process for preparing the ovule of the present invention comprising the steps of:
a) melting the fatty base at a temperature of 35-80°C;
b) adding colloidal silica to the molten fatty base while stirring;
c) precooling the mixture by slowly stirring until a mass temperature of 30-60°C is reached and then adding arasertaconazole mononitrate under vigorous stirring;
d) keeping stirring slowly until the mold temperature reaches 30-50°C;
e) filling the molds with the mass;
f) cooling the ovules in the molds; and
g) sealing the molds.

In other preferred embodiments, the temperature of step a) is 40-75 °C, the temperature of step c) is 35-55 °C and the temperature of step d) is 30-40 °C.

A further object of this application is to provide the ovule as defined in the present invention for treating vulvovaginal candidiasis. This aspect can be formulated as the use of an ovule as defined in the present invention for the manufacture of a medicament for the treatment of vulvovaginal candidiasis, as well as a method for the treatment of vulvovaginal candidiasis comprising administering an ovule of the present invention to a patient in need thereof.

### BRIEF DESCRIPTION OF THE DRAWINGS

FIG. 1 shows arasertaconazole plasma concentrations after vaginal administration of an ovule of arasertaconazole mononitrate 150 mg (A-150), as prepared in Example 1, in comparison to sertaconazole plasma concentrations after vaginal administration of an ovule of sertaconazole mononitrate 300 mg (S-300, Monazol). Plasma concentrations are expressed in ng/ml and time in hours (h).
FIG. 2 shows the same variables as in FIG. 1 but plasma concentrations are represented in logarithmic scale.
FIG. 3 shows arasertaconazole plasma concentrations after vaginal administration of ovules of arasertaconazole mononitrate 150 mg (A-150), 300 mg (A-300) and 600 mg (A-600) as prepared in Example 1. Plasma concentrations are expressed in ng/ml and time in hours (h).

### EXAMPLES

### Example 1: Ovules for vaginal application of arasertaconazole mononitrate 150 mg, 300 mg and 600 mg

The compositions of arasertaconazole mononitrate ovules are presented in Table 1.

**Table 1**

| ***Components*** | | ***Ovule 150 mg*** | | ***Ovule 300 mg*** | | ***Ovule 600 mg*** | |
|---|---|---|---|---|---|---|---|
| | | % w/w | mg | % w/w | mg | % w/w | mg |
| Arasertaconazole mononitrate (AM) | | 6.250 | 150.0 | 12.50 | 300.0 | 25.00 | 600.0 |
| Colloidal anhydrous silica (Aerosil®200) | | 3.000 | 72.0 | 3.00 | 72.0 | 3.00 | 72.0 |
| Components of fatty base | Coconut-hydrogenated glycerides + Glyceryl ricinoleate (Witepsol^{®} H19) | 45.375 | 1089.0 | 42.25 | 1014.0 | 36.00 | 864.0 |
| | Semi-synthetic glyceride (Suppocire^{®} NAi 50) | 45.375 | 1089.0 | 42.25 | 1014.0 | 36.00 | 864.0 |
| *TOTAL* | | *100%* | *2400. 0* | *100%* | *2400. 0* | *100%* | *2400. 0* |

### Example 2: Process for the preparation of ovules for vaginal application of arasertaconazole mononitrate 150 mg, 300 mg and 600 mg

The process for the preparation of 3 batches of 1000 ovules each is described below. Each batch corresponds to 150, 300 and 600 mg ovules as described in Example 1. Table 2 shows the amount of ovule components expressed in grams.

**Table 2**

| ***Components*** | | ***Ovule 150 mg*** | ***Ovule 300 mg*** | ***Ovule 600 mg*** |
|---|---|---|---|---|
| Arasertaconazole mononitrate (AM) | | 150 | 300 | 600 |
| Colloidal anhydrous silica (Aerosil^{®}200) | | 72 | 72 | 72 |
| Components of fatty base | Coconut-hydrogenated glycerides + Glyceryl ricinoleate (Witepsol^{®} H19) | 1089 | 1014 | 864 |
| | Semi-synthetic glyceride (Suppocire^{®} NAi 50) | 1089 | 1014 | 864 |
| *TOTAL* | | *2400* | *2400* | *2400* |

a) fatty base components were weighed and subject to a temperature around 42-48°C until the complete melt of the components;
b) colloidal anhydrous silica was weighed and, without stopping stirring, added to the molten fatty base by stirring with a turbo-stirrer;
c) stirring was stopped with the turbo-stirrer and the mass was precooled by stirring slowly until a temperature ranging 36-40°C was reached and then previously weighed AM was added using the turbo-stirrer;
d) finally, the turbo-stirrer was stopped and stirring was continued slowly until the mold temperature reached around 33-35°C;
e) the molds were filled with the mass;
f) the ovules were cooled in the molds; and
g) the molds were sealed.

Working in the temperature ranges pointed out in steps (a), (c), and (d), the same effect on the process is achieved.

### Example 3: Determination of Arasertaconazole mononitrate absorption levels after vaginal application of 150 mg, 300 mg and 600 mg ovules

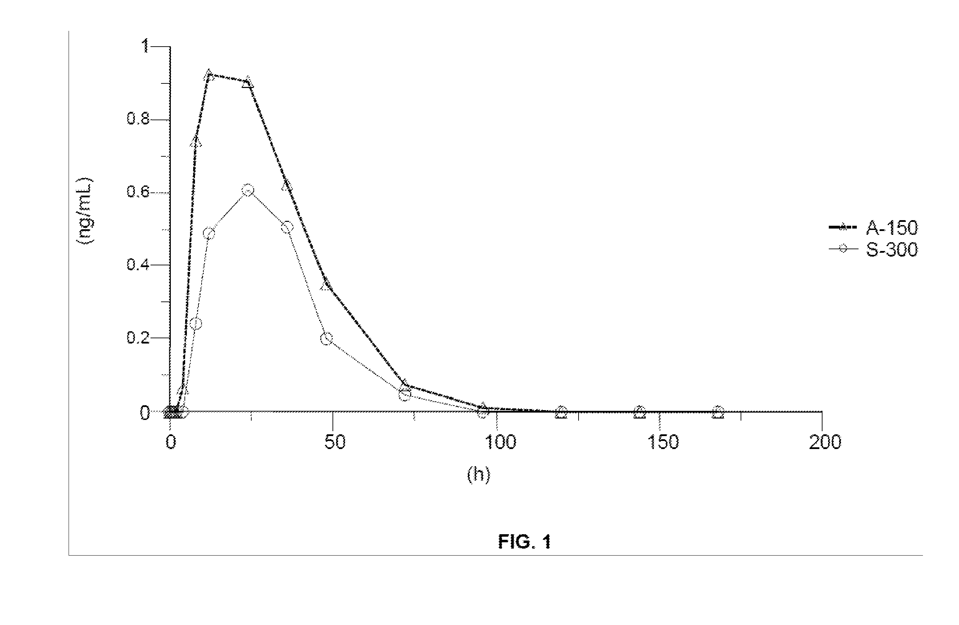

In a comparative clinical study involving women aged 18-65 years, the absorption of arasertaconazole was assessed after vaginal administration of AM 150, 300 and 600 mg ovules (Example 1) versus sertaconazole mononitrate (SM) 300 mg ovules (Monazol^{®}). Plasma levels were determined by HPLC (FIG. 1-3).

The HPLC conditions were the following:
Column: ChiraDex 4.0x250mm, 5 µm, Agilent
Injected Sample volume: 30 µL
Flow rate: 0.8 mL/min.
Mobile phase: Mixture of methanol and buffer with ammonium formiate.

The retention time for the R and S enantiomers of sertaconazole were about 7.4 and 9.1 minutes, respectively. In order to determine the plasmatic concentration of sertaconazole, the values obtained for the R and S enantiomers were added.

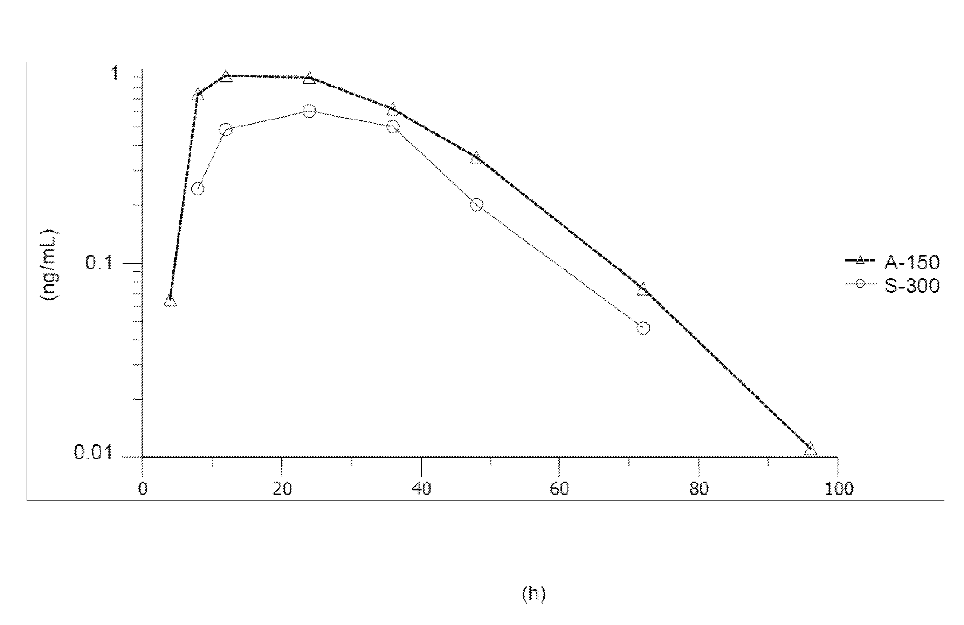

FIG. 1 shows that the levels reached with the AM 150 mg ovule (A-150) were higher than those reached with the SM 300 mg ovule (S-300). This means that the presence of the less active enantiomer of sertaconazole, (S)-sertaconazole, has a negative effect on the vaginal absorption of the most active enantiomer, i.e., arasertaconazole, in the SM ovule. Consequently, the AM 150 mg ovule is surprisingly and advantageously superior to the SM 300 mg ovule. If, in addition, the results are expressed on a logarithmic scale, arasertaconazole levels are also shown to be more persistent over time than those of sertaconazole (FIG. 2).

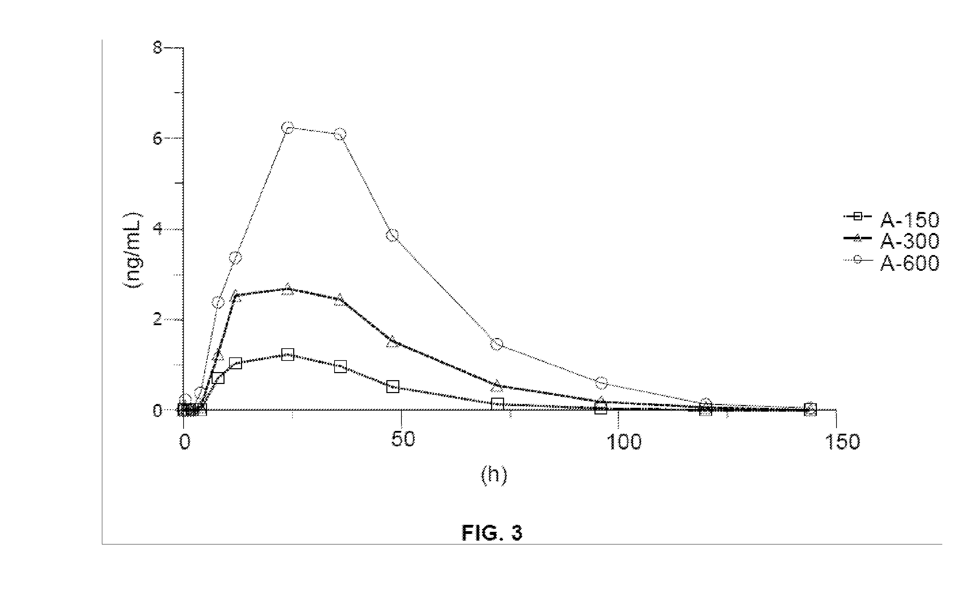

FIG. 3 clearly shows that plasma concentrations are dose dependent and, thus, dosage can be adjusted to the severity of the cases. Dose-dependent effects on plasma concentrations indicate that no saturation phenomenon appears to exist in the dose range studied.

## Claims

1. An ovule for vaginal administration of arasertaconazole mononitrate comprising 100 to 1000 mg of arasertaconazole mononitrate and a mixture of excipients which comprises:
(i) colloidal silica in the range from 0.5 to 5% by weight of the entire ovule;
and
(ii) fatty base in a sufficient range up to 100% by weight of the entire ovule.

2. The ovule according to claim 1 wherein the proportion of arasertaconazole mononitrate represents 3 to 50% by weight of the entire ovule.

3. The ovule according to claim 1 wherein the amount of arasertaconazole mononitrate comprises 150 to 600 mg.

4. The ovule according to claim 3 wherein the proportion of arasertaconazole mononitrate represents 5 to 30% by weight of the entire ovule.

5. The ovule according to claim 1 wherein colloidal silica is present in an amount of 3% by weight of the entire ovule.

6. The ovule according to claim 1 wherein colloidal silica is in anhydrous form.

7. The ovule according to claim 1 wherein the fatty base is selected from the group consisting of: triglyceride, monoglyceride and diglyceride esters of C₈ - C₂₀ fatty acids, and the mixtures thereof.

8. A process for preparing the ovule of claim 1 which comprises the steps of:
a) melting the fatty base at a temperature of 35-80°C;
b) adding colloidal silica to the molten fatty base while stirring;
c) precooling the mixture by stirring slowly until a mass temperature of 30-60 °C is reached and then adding arasertaconazole mononitrate under vigorous stirring;
d) keeping stirring slowly until the mold temperature reaches 30-50°C;
e) filing the molds with the mass;
f) cooling the ovules in the molds; and
g) sealing the molds.

9. The process according to claim 8 wherein the temperature of step a) is 40-75 °C.

10. The process according to claim 8 wherein the temperature of step c) is 35-55 °C.

11. The process according to claim 8 wherein the temperature of step d) is 30-40 °C.

12. An ovule as defined in any one of claims 1 to 7 for treating vulvovaginal candidiasis.
